# EUROPEAN PATENT SPECIFICATION

(11) **EP 3 691 695 B1**
(45) Date of publication and mention of the grant of the patent: **17.04.2024**
(21) Application number: 18870535.4
(22) Date of filing: 01.10.2018
(51) Int. Cl.: A61K 48/00, C12N 5/077, C12N 5/0789, C12N 5/10, C12N 15/49, C12N 15/63, C12N 15/867, C12N 5/0775

(54) **METHOD OF PRODUCING NATURE IDENTICAL CARTILAGE TISSUE VIA GENE DELIVERY**
VERFAHREN ZUR HERSTELLUNG VON NATURIDENTISCHEM KNORPELGEWEBE MITTELS GENTRANSFER
PROCÉDÉ DE PRODUCTION DE TISSU CARTILAGINEUX IDENTIQUE À UN TISSU NATUREL PAR L'INTERMÉDIAIRE D'UNE ADMINISTRATION DE GÈNE

(30) Priority: 03.10.2017 TR 201714838
(43) Date of publication of application: 12.08.2020
(73) Proprietor: Yeditepe Üniversitesi, Istanbul (TR)
(72) Inventor: TORUN KÖSE, Gamze, 34755 Istanbul (TR); TELCI, Dilek, 34755 Istanbul (TR); ÇALIKOGLU, Ayse Ceren, Atasehir/Istanbul (TR)
(74) Representative: Dericioglu, E. Korhan
(86) International application number: PCT/TR2018/050536
(87) International publication number: WO 2019/083482

(56) References cited:
- WO-A1-2015/035395
- CALIKOGLU KOYUNCU A. C. ET AL: "Cartilage tissue engineering on macroporous scaffolds using human tooth germ stem cells : Cartilage tissue engineering on macroporous scaffolds", JOURNAL OF TISSUE ENGINEERING AND REGENERATIVE MEDICINE, vol. 11, no. 3, 2 January 2015 (2015-01-02), pages 765-777, XP055800150, US ISSN: 1932-6254, DOI: 10.1002/term.1975 Retrieved from the Internet: URL:https://api.wiley.com/onlinelibrary/td m/v1/articles/10.1002%2Fterm.1975>
- DMITRY NURMINSKY ET AL: "Transglutaminase 2 regulates early chondrogenesis and glycosaminoglycan synthesis", MECHANISMS OF DEVELOPMENT, ELSEVIER SCIENCE IRELAND LTD, IE, vol. 128, no. 3, 25 November 2010 (2010-11-25), pages 234-245, XP028156419, ISSN: 0925-4773, DOI: 10.1016/J.MOD.2010.11.007 [retrieved on 2010-12-01]
- OLIVEIRA JOÃO T. ET AL: "Novel Melt-Processable Chitosan-Polybutylene Succinate Fibre Scaffolds for Cartilage Tissue Engineering", JOURNAL OF BIOMATERIALS SCIENCE. POLYMER EDITION., vol. 22, no. 4-6, 1 January 2011 (2011-01-01), pages 773-788, XP055800166, NL ISSN: 0920-5063, DOI: 10.1163/092050610X494604 Retrieved from the Internet: URL:https://www.tandfonline.com/doi/pdf/10 .1163/092050610X494604>
- Kristen A. JOHNSON: "External GTP-bound Transglutaminase 2 is a molecular switch for chondrocyte hypertrophic differentiation and calcification", THE JOURNAL OF BIOLOGICAL CHEMISTRY, vol. 280, no. 15, 3 February 2005 (2005-02-03), pages 15004-15012, XP055617354,
- WOO-SHIN KIM et al.: "Transglutaminase 2 regulates osteoclast differentiation via a Blimp 1 -dependent pathway", NATURE SCIENTIFIC REPORTS, vol. 7, no. 10626 6 September 2017 (2017-09-06), pages 1-17, XP055617363,
- IREM AYSE KANNECI ALTINISIK et al.: "In vitro evaluation of PLLA/PBS sponges as a promising biodegradable scaffold for neural tissue engineering", TURK J BIOL, vol. 41, 13 April 2017 (2017-04-13), pages 734-745, XP055617365,
- CORINNE NIGER et al.: "Induction of chondrogenic differentiation in mesenchymal stem cells by TGF-beta cross-linked to collagen-PLLA [poly(L-lactic acid)] scaffold by transglutaminase 2", BIOTECHNOL LETT, vol. 35, no. 12, 27 July 2013 (2013-07-27) , pages 1-14, XP055617373,

## Description

### Field of the Invention

The present invention relates to production of cartilage tissue that is similar to the natural one cultivating rat mesenchymal stem cells to which tissue transglutaminase gene (variant 2) is delivered by lentiviral transduction, on three dimensional poly(1,4-butylene succinate)/poly(L-lactic acid) tissue scaffolds for use in cartilage tissue defects and tissue regeneration.

### Background of the Invention

The cartilage tissue functions as a semi-carrier in the body and has a resilient and smooth matrix compared to the bone tissue. Since the cartilage tissue does not contain blood vessels unlike the bone tissue, it exchanges substances through the tissue matrix (passive diffusion). Due to the absence of blood and lymphatic system, the cartilage tissue cannot regenerate itself easily when injured. Especially in cases of major defects, surgeries and even implants are required.

Due to the insufficiency of the treatment methods used today, the best solution for the damaged cartilage tissue appears as cartilage tissue engineering. The three important factors in cartilage tissue engineering are the cells, tissue scaffolds, and differentiation factors. Although chondrocytes (cartilage cells) are the primary source of cells, they are not preferred because the intact healthy tissue is damaged during isolation of the cells. Instead, generally mesenchymal stem cells (MSCs) with their potential to differentiate into cartilage cells are used. These cells can be extracted from many parts of the body, such as the bone marrow, cartilage tissues, teeth, and adipose tissues, however; the MSCs extracted from the bone marrow has the highest potential of differentiation.

In order to ensure proper cartilage tissue regeneration, cells must be cultivated on three-dimensional tissue scaffolds. There are many natural and synthetic materials used for this purpose. While the natural materials offer a more physiologically biocompatible environment than the synthetic materials, they may lead to immune responses since they may contain various antigens. In this respect, synthetic materials are more suitable for cartilage tissue engineering. Poly(α-hydroxyacids) such as poly(glycolic acid) (PGA), poly(lactic acid) (PLA) and poly(lactic-co-glycolic acid) (PLGA) are synthetic polymers widely used in the cartilage tissue engineering. These materials have been used since the 1990s as they are approved by the US Food and Drug Administration (FDA) and they are biocompatible and biodegradable. There are numerous studies in the literature that show the role of PLLA on the formation of cartilage tissue. Poly(ε-caprolactone) (PCL), poly(urethane) (PUR), poly(propylene fumarate) (PPF), poly(1,4-butylene succinate) (PBSu) and poly(ethylene glycol) (PEG) are the examples of other synthetic materials that are used. PBSu, which is a biomaterial that is more ductile and more flexible than PLLA, is also a biocompatible and biodegradable synthetic polymer. Since PBSu is a more recently discovered material than PLLA, there are a few studies in the literature showing that it contributes to the cartilage tissue formation. In addition to the fact that both of these materials have mechanical properties suitable for tissue engineering in terms of toughness and flexibility, it was found that the by-products resulting from their degradation are harmless to health. Because PLLA is tougher than PBSu, and PBSu is more flexible than PLLA, it is aimed to improve the mechanical properties of the tissue scaffold that will be formed and thus further support the tissue formation using the blend of these two materials.

Various growth and differentiation factors such as Insulin-like Growth Factor-1 (IGF-1) and Transforming Growth Factor-Beta (TGF-β) are used to increase the differentiation potential of the cells on the tissue scaffolds. While these factors can be added directly to the culture medium, they can also be entrapped within the tissue scaffolds or delivered to the cells by means of gene delivery. All of these three methods are used effectively in the field of cartilage tissue engineering. In a study to enhance TGF-β binding on the scaffolds and hence to enable cell differentiation, the tissue scaffolds were coated with TG2 which is a cross-linking protein. As a result, more TGF-β was successfully cross-linked onto the tissue scaffolds by TG2 leading to increased TGF-β activation and cell differentiation. In addition, TG2 is an extracellular matrix binding protein and functions as a bridge between integrin and fibronectin, allowing the cell to bind strongly to its own extracellular matrix. The strong connection between the cell and its matrix is desirable in terms of new tissue formation or tissue regeneration.

Among the four variants (or isoforms) of TG2 in the literature, the type having the highest gene expression in human tissues is variant 1 (V1). This is followed by variant 2 (V2), variant 4 (V4) and variant 3 (V3), respectively. These variants formed as a result of alternative splicing are proposed to have higher protein activity. In the literature, there is no study where any of these variants is used in cartilage tissue engineering.

In cartilage tissue engineering studies conducted so far, the ideal tissue scaffold that enables the formation of the most natural identical tissue has not yet been determined. In addition, tissue engineering studies generally suffers from the failure of fast degradation of the polymeric tissue scaffolds leading to the detachment of the cells from the surface of the degraded scaffolds. As a solution to this problem, there is a need for factors that will reinforce the cell-tissue scaffold interaction.

### Summary of the Invention

The objective of the present invention is to cultivate the cells to which gene delivery is performed using lentiviruses, on polymeric tissue scaffolds, and then to use them in cartilage tissue defects and tissue regeneration.

Another objective of the invention is to prevent the cells from detaching themselves from the polymeric scaffold by establishing the ideal tissue scaffold that will enable the most nature identical tissue formation.

### Detailed Description of the Invention

**"Method of Producing Nature Identical Cartilage Tissue via Gene Delivery"** developed to fulfill the objectives of the present invention is illustrated in the accompanying figures, in which;
- **Figure 1.**: is the map of the hTGM2(V2) plasmid vector. (pLV[Exp]-Bsd-EF1A>hTGM2[NM_198951.1], bp: base pair)
- **Figure 2.**: shows the binding regions of the sequencing primers on the hTGM2(V2) plasmid vector. (hTGM2 (V2) sequence: 3168-4814, Primer F: primer forward, Primer R: primer reverse)
- **Figure 3.**: is the sequencing chromatogram of the first portion of the hTGM2 (V2) gene in plasmid vector. (The regions within the boxes denote the parts overlapping with the original sequence.)
- **Figure 4.**: shows the sequencing chromatogram of the second portion of the hTGM2 (V2) gene in plasmid vector. (the regions within the boxes denote the segments overlapping with the original sequence.)
- **Figure 5.**: are the BLAST results of the original and plasmid vector hTGM2(V2) gene sequences. (first segment)
- **Figure 6.**: are the BLAST results of the original and plasmid vector hTGM2(V2) gene sequences. (second segment)
- **Figure 7.**: representation of the TG2 (V2) expression in transduced cells in the Real-Time PCR graph at mRNA level (a) and in the Western Blot graph (b) at the protein level. (+Trans: Transduced cells, -Trans: Non-transduced cells, Control: TG2 enzyme)
- **Figure 8.**: image of the GFP expression in the MSCs under fluorescent microscope 18 hours (a), 4 days (b), 7 days (c) after transduction.
- **Figure 9.**: shows the immunofluorescence images of the transduced MSCs (a) and non-transduced control MSCs (b) after being labeled by collagen type 2 at the end of 14 days. (lens: 20X, scale: 100 µm) (Green: collagen type 2; blue: DAPI) (Chondrogenic induction is not performed)
- **Figure 10.**: shows the bright-field microscope images of the transduced MSCs (a) and non-transduced control MSCs (b) after being stained by Alcian Blue at the end of 14 days. (lens: 10X, scale: 200 µm) (Chondrogenic induction is not performed)
- **Figure 11.**: shows the bright-field microscope images of the transduced MSCs (a) and non-transduced control MSCs (b) after being stained by Alizarin Red at the end of 14 days. (lens: 5X, scale: 500 µm) (Chondrogenic induction is not performed)
- **Figure 12.**: is the scanning electron micrograph of the PBSu/PLLA tissue scaffolds.
- **Figure 13.**: is the fluorescent microscope image of the cells expressing EGFP seeded on PBSu/PLLA tissue scaffolds. (lens: 5X, scale: 500 µm)
- **Figure 14.**: is the graphical representation of the MTS analysis of the cells seeded on the scaffolds. (Initial cell seeding density: 50000 cells / tissue scaffold.) (2mL: Transduced rBMSCs, MSC: rBMSCs, CH: chondrocyte; (-): no chondrogenic induction, (+): chondrogenic induction)
- **Figure 15.**: is the fluorescent microscope (Carl Zeiss Axio Vert.A1) image of transduced (a, b) and non-transduced cells (c, d) after being labeled by TG2 antibody at the end of 21 days. (Green: TG2; blue: DAPI) (a, c: Samples on which chondrogenic differentiation is not applied; b, d: Samples on which chondrogenic differentiation is applied; e: Empty matrix) (lens: 10X, scale: 50 µm)
- **Figure 16.**: is the confocal microscope (Carl Zeiss LSM 700) image of the transduced (a) and non-transduced cells (b) after being labeled by the antibodies of cartilage specific proteins at the end of 21 days. (Green: collagen type 2; blue: DAPI; red: aggrecan; a, b: Samples on which chondrogenic differentiation is not applied; c: control cartilage cells) (lens: 20X; scale a: 20 µm, b-c: 50 µm)

The components shown in the figures are each given reference numbers as follows:
**1.** RSV Promoter: Rous sarcoma virus (RSV) promoter
**2.** Δ5' LTR: HIV-1 truncated 5' LTR
**3.** Ψ: HIV-1 psi packaging signal
**4.** RRE: HIV-1 Rev response element
**5.** cPPT: Central polypurine tract
**6.** EF1A: Elongation Factor 1A promoter
**7.** Kozak
**8.** *hTGM2[NM_198951.1]*
**9.** *WPRE: Woodchuck hepatitis virus posttranscriptional regulatory element*
**10.** *PGK: Mouse phosphoglycerate kinase promoter*
**11.** *Blasticidin: Blasticidin resistance gene*
**12.** ΔU3/3' LTR: HIV-1 truncated 3' LTR
**13.** *SV40 early pA: SV40 early polyadenylation signal*
**14.** *Ampicillin: Ampicillin resistance gene*
**15.***pUC ori: pUC replication origin*

***PF1.*** Primer Forward 1
***PF2.*** Primer *Forward 2*
***PR1.*** Primer *Reverse 1*
***PR2.*** Primer *Reverse 2*

The inventive method for producing a nature identical cartilage tissue for use in cartilage tissue defects and tissue regeneration, comprises the steps of
- cultivating the mesenchymal stem cells (MSC) extracted from rat bone marrow in a sterile environment in cell culture dishes, seeding the cultivated cells to cell culture dishes after being passaged,
- in the meantime, amplifying the psPAX2 (lentiviral packaging plasmid), pMD2.G (lentiviral envelope plasmid) and pTGM2(V2) (expression plasmid) plasmids by using *E.coli* bacteria,
- confirming the sequence of hTGM2 (V2) gene in pTGM2(V2),
- producing lentiviruses containing pTGM2 gene by delivering psPAX2, pMD2.G, or pTGM2(V2) plasmids to HEK293T cells in triplets,
- when the mesenchymal stem cells (MSC) are at approximately 70% confluency, performing transduction process of the mesenchymal stem cells by adding lentiviruses onto the mesenchymal stem cells (MSC) such that 2 ml of lentivirus is added per culture dish,
- incubating the mesenchymal stem cells (MSC) in the CO₂ incubator for about 24 hours,
- at the end of the incubation, performing PBS washing and then cell seeding in a normal medium and proceeding with the usual cell culture,
- incubating the transduced cells in a medium containing Blasticidin antibiotic for 10 days and eliminating the cells that has not taken up the transgene,
- proving that living cells have successfully acquired the gene,
- preparing poly(1,4-butylene succinate)/ poly-L-lactide (PLLA) (PBSu/PLLA) polymer tissue scaffolds by salt leaching technique,
   o weighing the poly(1,4-butylene succinate) (PBSu) and poly-L-lactide (PLLA) polymers at a ratio of 1:1 and dissolving them in dichloromethane to make 6% solutions,
   o mixing the prepared polymer solution with NaCl salts (300-500 microns in size) until it acquires a paste-like form and then pouring it into the 10 cm glass petri dishes coated with aluminum foil,
   ∘ one day later, taking the polymers out of the glass dishes and dialyzing them in distilled water for 2 days,
   o freezing and lyophilizing the polymers at -80°C,
   o cutting the polymers in the form of discs with a diameter of 7 mm,
   o sterilizing the cut discs in 70% ethanol solution in the refrigerator for 2 hours,
   o then washing with distilled water at least three times,
   o pouring medium on the polymer in the dishes and incubating it in CO₂ incubator overnight,
   o then, upon discarding the medium, obtaining sterile tissue scaffold,
- seeding cells in each PBSu/PLLA polymer tissue scaffold such that there will be 50000 cells per each scaffold,
- after waiting for adhesion of the cells to the tissue scaffolds (about 6 hours), adding medium onto the culture dishes and characterizing the cells,
- obtaining the nature identical cartilage tissue.

Within the scope of the invention, rat mesenchymal stem cells to which tissue transglutaminase gene (variant 2) is delivered by lentiviral transduction, are cultivated and differentiated on three dimensional poly(1,4-butylene succinate)/poly(L-lactic acid) tissue scaffolds. Since PLLA is tougher than PBSu, and PBSu is more flexible than PLLA, it is aimed to provide a more ideal tissue engineering scaffold in terms of mechanical properties using a blend of these two materials. Tissue transglutaminase gene delivery to the cells is planned to increase the adhesion of the cells onto tissue scaffolds and to provide a more rapid and ideal tissue formation. The nature identical cartilage tissue obtained by cultivating the cells to which gene delivery is performed using lentivirus, on the polymeric tissue scaffolds is used in cartilage tissue defects and tissue regeneration within the scope of genetic engineering, bioengineering and biotechnology fields. Since there is no transduction method including this gene in the literature search, the TGM2 (V2) gene delivery method used in this study has a novelty. At the same time, the product obtained by cultivating the MSCs containing this gene on polymeric tissue scaffolds is also original.

In the method of producing nature identical cartilage tissue according to the present invention, the mesenchymal stem cells (MSCs), extracted from rat bone marrow in a sterile environment, are cultivated in cell culture dishes. The cultivated cells, after being passaged once, are seeded in 6-well plates. At the same time, lentiviral packaging, envelope, and expression plasmids, namely psPAX2, pMD2.G, or pTGM2 (V2) respectively are amplified in E.coli, and then, the plasmid vector containing the hTGM2 (V2) gene is sent to sequencing to confirm the accuracy of the sequence of the gene it contains. Lentiviral packaging plasmids carry out the process of lentivirus packaging after the plasmid is transferred into the cell. There is no external involvement except that these three plasmids (packaging, envelope, and expression) need to be given to the cell at the same time so that viruses can spontaneously be formed within the cell. After confirmation of the sequence, lentiviruses carrying pTGM2 plasmid are produced by delivering psPAX2, pMD2.G, or pTGM2(V2) plasmids to HEK293T cells in triplets. If any errors are detected in the sequence, the plasmid isolation process should have to be repeated before starting the actual experiments. Therefore, this confirmation is a necessary process. When the mesenchymal stem cells (MSC) are at about 70% confluency, lentiviruses are added onto the mesenchymal stem cells (MSC) such that 2 ml of lentivirus is added to each well of the six-well plates (transduction process is performed) and the cells are incubated in CO₂ incubator for 24 hours. At the end of this period, PBS washing is performed once and then normal medium is added and the usual cell culturing is continued. The transduced cells are incubated in a medium containing Blasticidin antibiotic for 10 days and the cells that have not taken up the transgene are removed from the medium. Due to the Blasticidin-resistance gene found in the pTGM2 (V2) and pEGFP plasmids, the cells to which these plasmids are successfully transferred, are able to survive in a medium containing the antibiotic Blasticidin. The cells which are still alive at the end of this period (10 days) are subjected to protein (Western Blot) and gene expression (Real-Time PCR) tests to prove that they have successfully received the gene. In addition, Collagen Type-2 immunostaining and Alcian Blue staining are performed to examine whether the transduced cells are spontaneously differentiated. MTS analysis is performed to measure cell viability.

The poly(1,4-butylene succinate) / poly-L-lactide (PLLA) (PBSu/PLLA) (1:1) tissue scaffolds used as polymer tissue scaffolds are prepared by using salt leaching technique. In the process of preparing these tissue scaffolds, firstly, the polymers are weighed at a ratio of 1:1 and dissolved in dichloromethane to make 6% solutions. The polymer solution is mixed with NaCl salts (300-500 microns in size) until it acquires a paste-like form and then poured into the 10 cm glass petri dishes coated with aluminum foil. The next day, the polymers are removed from the glass dishes, stored in distilled water for 2 days to dissolve the salts, and finally, the polymers are frozen and lyophilized at -80°C and then cut into discs of 7 mm diameter. The cut discs are sterilized in the refrigerator in 70% ethanol solution for 2 hours, and then washed 3 times with distilled water, and after adding the medium, they are incubated overnight in the CO₂ incubator. The next day, the medium is discarded using a pipette, cells are seeded as 50,000 cells per sample. The samples are subjected to cell viability and differentiation tests for 14 and 21 days. Cell viability tests are carried out to test whether the cells are able to maintain their viability and proliferate at the end of the above-mentioned procedures. The differentiation tests are conducted for observing whether the cells produce cartilage specific proteins and acquire features similar to a natural tissue. If the cells lose their viability, cannot proliferate or cannot differentiate after the processes, it will be concluded that the cells are not suitable to be used in this study.

The nature identical cartilage tissue production method of the present invention is original both in terms of the idea of applying tissue engineering by using tissue transglutaminase (variant 2) gene and the product that is produced, because there is no tissue engineering product produced by using this gene in the literature.

### EXPERIMENTAL STUDIES

### Sequencing hTGM2(V2) Gene in pTGM2(V2) Plasmid Vector

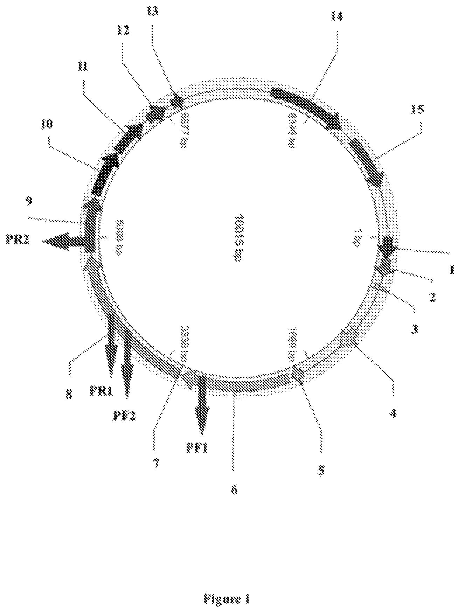

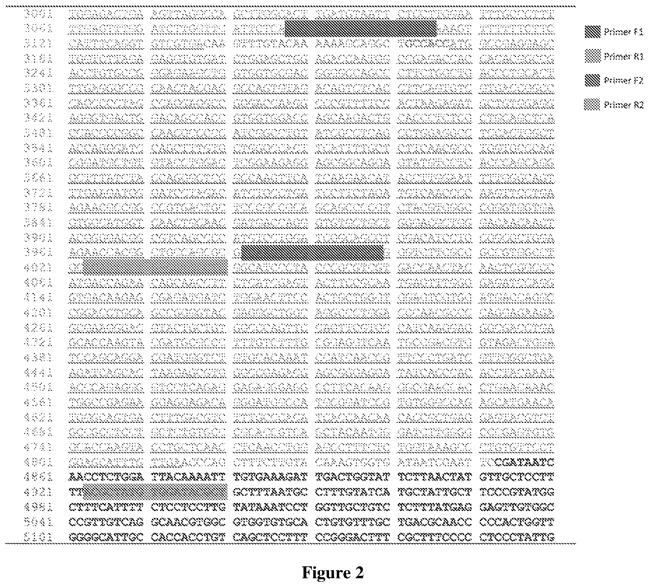

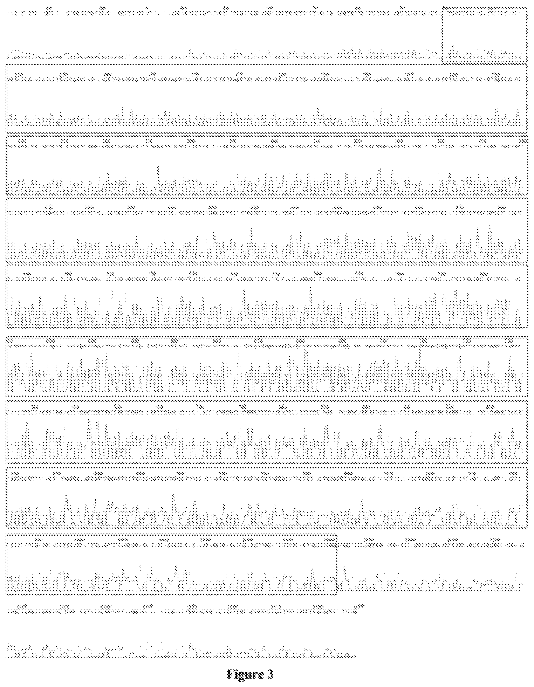

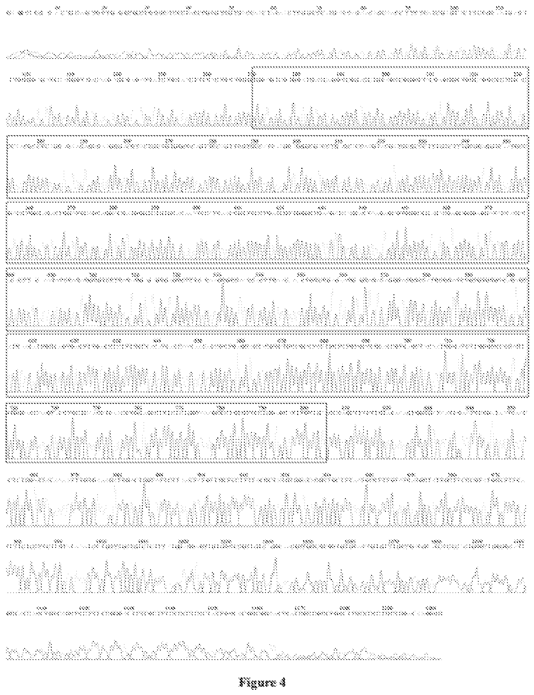

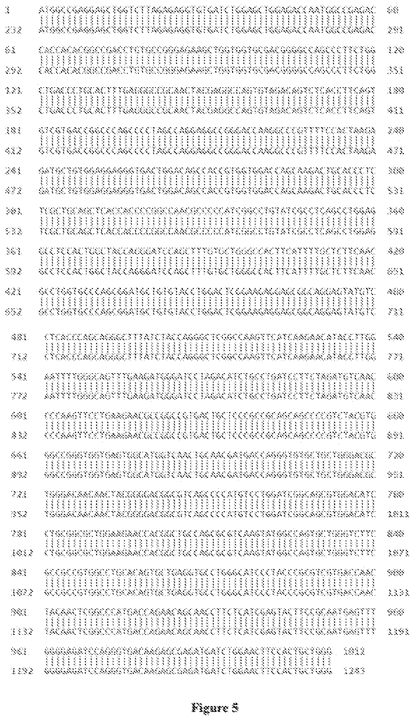

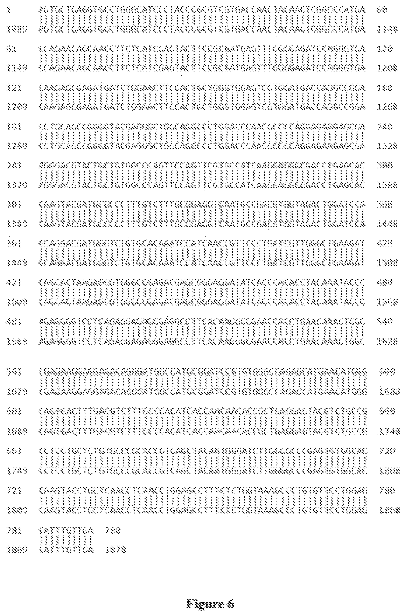

Sequencing is performed for proving the compatibility of the hTGM2(V2) gene that is inserted into the plasmid vector with the original sequence. The map of the plasmid is shown in Figure 1. In figure 2, the binding regions of the sequencing primers on the plasmid vector are marked. hTGM2(V2) sequence is between the nucleotides 3168 and 4814. The hTGM2(V2) gene inserted into the vector is sequenced in two parts because it is too long. Chromatograms of the sequenced parts are shown in Figure 3 and 4. Compatibility of the sequenced parts with the original hTGM2(V2) gene are proven by the BLAST results in Figure 5 and 6.

### TGM2(V2) Gene Delivery and Cell Characterization

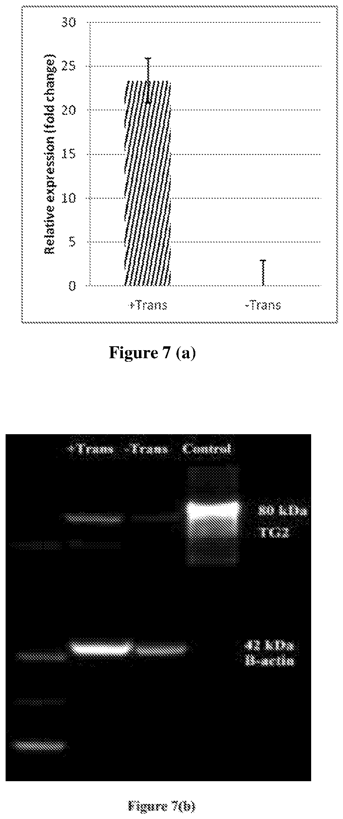

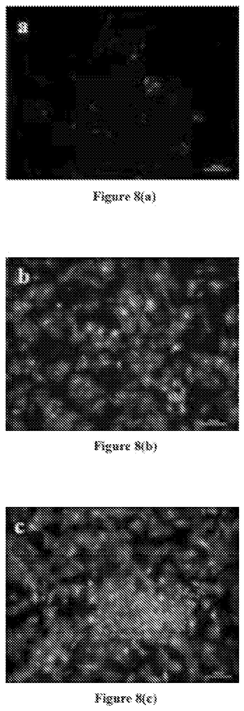

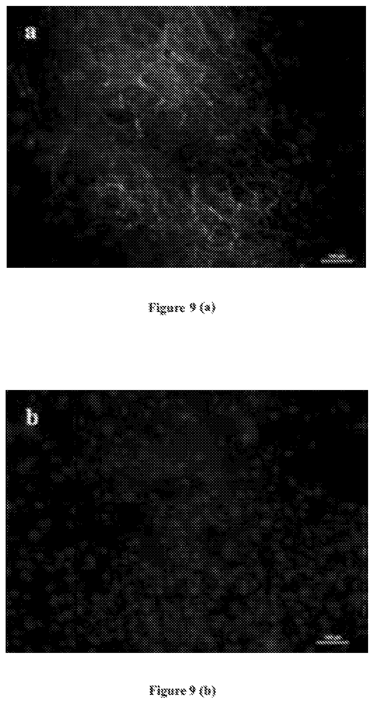

Gene and protein expressions are analyzed for testing whether the gene delivery to the transduced cells has been performed properly. As a result of Real-Time PCR, significant increase in the TGM2(V2) gene expression is observed (Figure 7a). Similarly, Western Blot results show that the protein expression increases as well (Figure 7b). The result of flow cytometry carried out at the end of second passage indicates that the MSCs transduced with EGFP as the control shows 99.56% positivity. This means that almost all of the transduced MS cells express the gene, i.e. the transduction is successful. As indicated in Figure 8 as well, the EGFP expression in the transduced cells exhibits an increase day by day. Collagen type 2 staining is carried out to see if there is a spontaneous chondrogenic differentiation in the cells incubated in the growth medium for 14 days following the transduction. As seen in Figure 9, while collagen type 2 expression is not detected in the cells to which gene delivery is not performed a, b, (Figure 9b); after TGM2(V2) transduction, collagen type 2 formation is observed in the cells (Figure 9a).

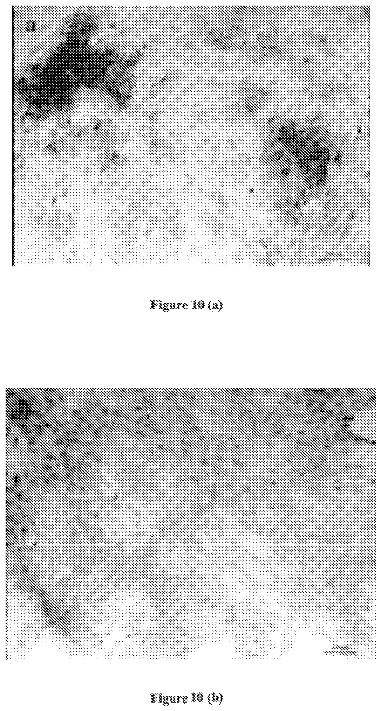

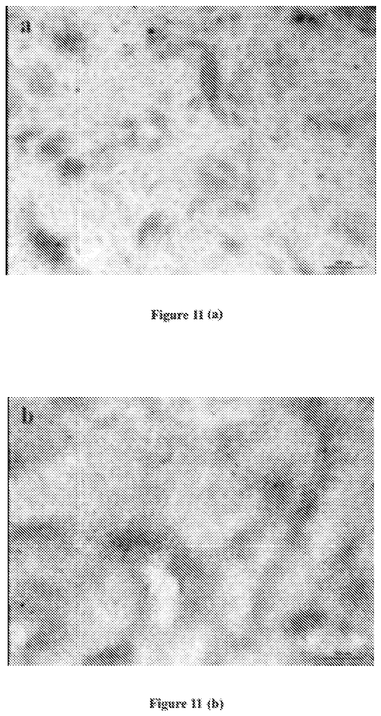

In order to support the above given findings, the extracellular matrix deposition is examined by Alcian Blue staining on the 14^{th} day. Alcian Blue stains the cartilage tissue specific sulfated glycosaminoglycans (s-GAG) blue. The blue color intensity is directly proportional to differentiation. Different from the control cells, s-GAG deposition is observed as groups in the transduced cells (Figure 10a, b). As a result of the Collagen type 2 and Alcian Blue staining, it can be observed that MSCs to which TGM2(V2) gene is delivered via lentiviral transduction, can differentiate into chondrocytes without the addition of induction medium. Alizarin Red staining is carried out to investigate presence of osteogenic differentiation in the transduced cells. Calcium phosphate crystals accumulated in the extracellular matrix is an indication of osteogenic differentiation. Alizarin red binds to the calcium phosphate crystals and stains them red. Figure 11 a and b show that the transduced cells store calcium phosphate although being less than the control cells.

### Characterization of PBSu/PLLA Tissue Scaffolds with or without Cells

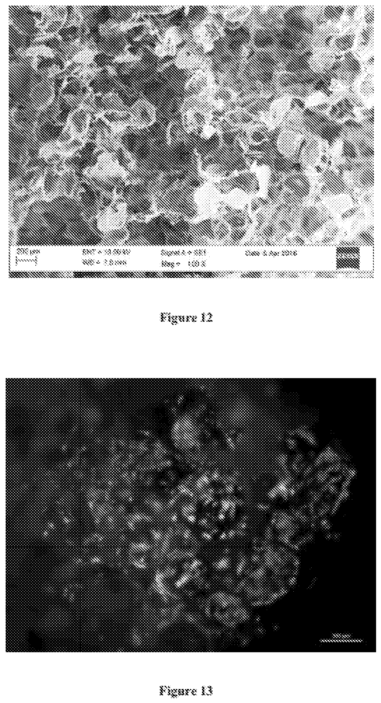

The polymer solutions are poured into 10 cm glass dishes. Approximately 66 pieces of 7 mm diameter polymer discs are obtained from these dishes. In order to observe the surface structures of the tissue scaffolds, scanning electron microscopy is performed. The porous structures of the scaffolds are shown in Figure 12. These pores increase the surface areas of the tissue scaffolds to a great extent, and in addition, they enable the nutrients present in the medium to be distributed better within the tissue scaffold and to reach the cells more easily. Figure 12 shows the scanning electron micrograph of the PBSu/PLLA tissue scaffolds. After seeding the cells transduced with EGFP, the samples are examined by fluorescence microscopy in order to demonstrate the cell adhesion capacity of the tissue scaffolds. The cells are observed to adhere and spread into the pores in the tissue scaffolds sturdily (Figure 13).

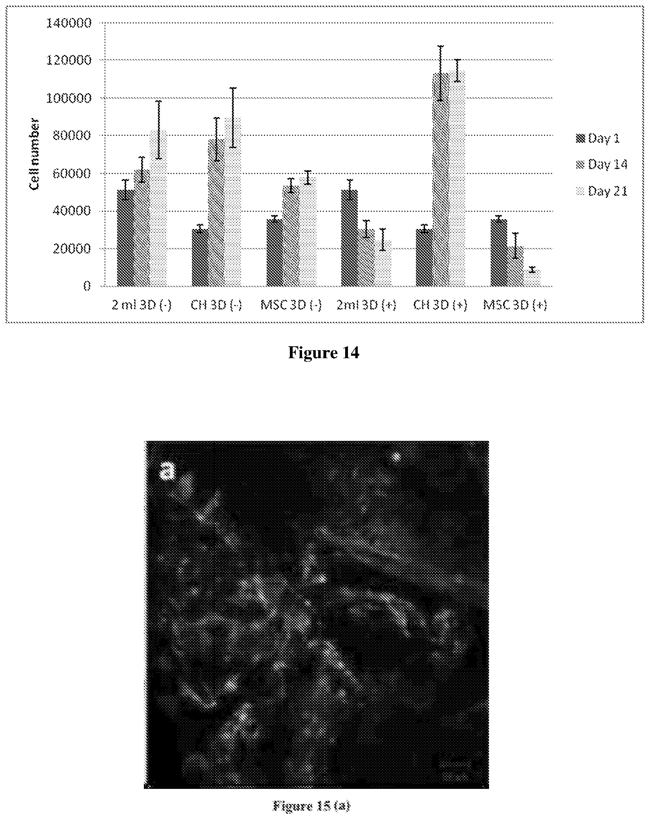

MTS analysis is performed on days 1, 14 and 21 in order to test cell viability on the tissue scaffolds. The result of this analysis is shown in Figure 14. When the cells which are not induced chondrogenically are compared with each other, it can be observed that the transduced cells have a better proliferation profile than the non-transduced MSCs. This result proves the theory that TGM2(V2) transduction increases adhesion capacity of the MSCs to the tissue scaffolds. In cells where chondrogenic induction is applied, it is expected to observe a decrease in the number of cells, because when the cells differentiate their rate of proliferation decreases.

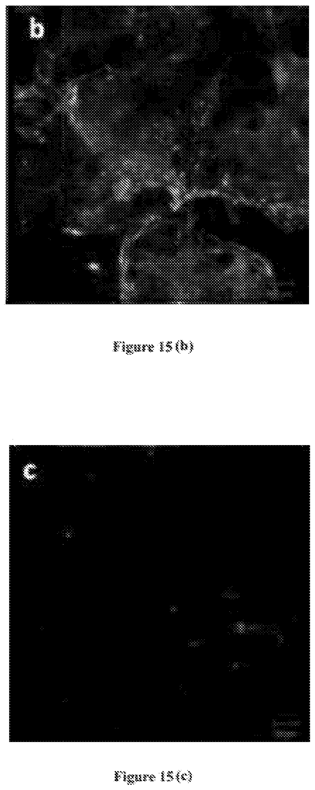

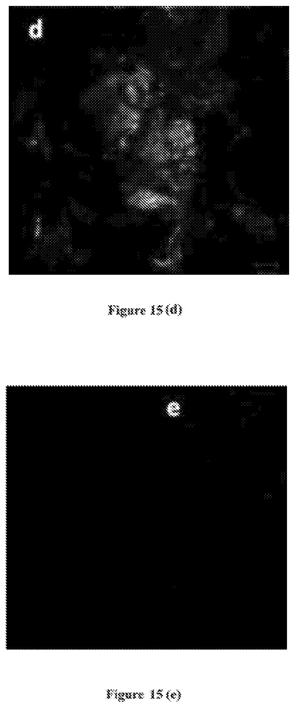

TG2 immunostaining is performed at the end of 21 days in order to observe whether the transduced cells maintain TG2(V2) deposition on the matrix. As a result it is proven that the transduced cells can deposit TG2 into the matrix (Figure 15a, b). As expected, TG2(V2) deposition is not observed on the non-transduced cells which are not chondrogenically induced (Figure 15c). A slight TG2(V2) deposition is observed on the non-transduced cells which are chondrogenically induced (Figure 15d). Based on these results, it is suggested that TGM2 transduced cells can be used in cartilage tissue engineering even without chondrogenic differentiation.

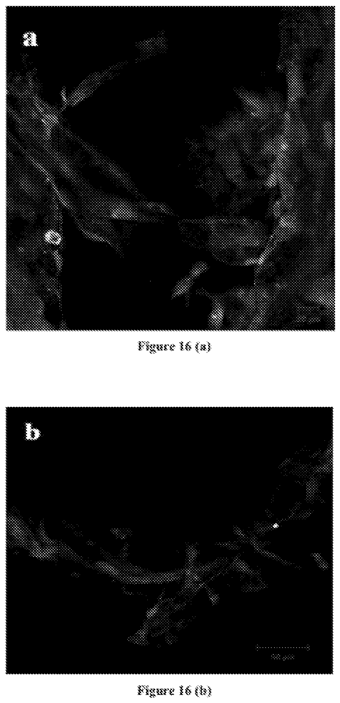

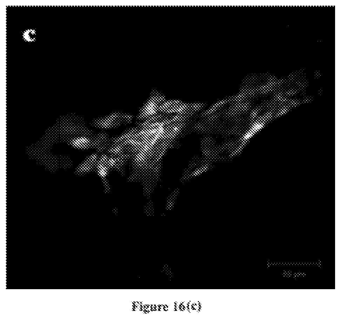

Collagen type 2 and aggrecan proteins are cartilage specific proteins and are used as markers of chondrogenic differentiation. In other words, the presence of collagen type 2 and aggrecan proteins in the environment indicate cartilage cell or tissue formation. It is shown in Figure 16 that the transduced cells can spontaneously produce these cartilage specific proteins on the polymer tissue scaffolds as well. It can be observed that the transduction process enhances the ability of cells to synthesize cartilage-specific proteins especially when compared to the non-transduced cells.

With the experimental studies conducted within the scope of developing the nature identical cartilage tissue production method of the present invention, it is suggested that PBSu/PLLA tissue scaffolds can be used in cartilage tissue engineering studies. In addition, TGM2 (V2) gene delivery to the MSCs enables both a stronger cell adhesion to the tissue scaffold and differentiation into cartilage without using induction. The nature identical cartilage tissue produced within the scope of the invention by use of TGM2(V2)-delivered MSCs with PBSu/PLLA tissue scaffolds is a suitable and specific candidate for cartilage tissue regeneration.

A few cartilage tissue engineering study performed using PBSu, which is a synthetic polymer that may be considered new in the literature, draws attention. Assuming this may be caused by the brittleness of the PBSu polymer, usage of this polymer in combination with other synthetic polymers such as PLLA, which has good mechanical properties, will solve this problem and contribute to the production of the ideal tissue scaffold that will provide the most nature identical tissue formation. In this study, MSCs to which TGM2(V2) gene is delivered by lentiviral method are enabled to adhere onto the PBSu/PLLA tissue scaffolds easily and thus differentiate into cartilage due to the facts that TG2 has extracellular matrix crosslinking feature and functions as a cell adhesion molecule. At the same time, the TGM2(V2) transduced cells are shown to be able to spontaneously differentiate into chondrocytes without using any differentiation factors that are both expensive and have short half-life in the body. The cell tissue scaffold developed in the scope of the invention is proposed as a suitable candidate for future cartilage tissue engineering studies.

## Claims

1. A production method for preparing the most nature identical cartilage tissue for use in cartilage tissue defects and cartilage tissue regeneration, **characterized by** the steps of
- cultivating the mesenchymal stem cells (MSC) extracted from rat bone marrow in a sterile environment in cell culture dishes,
- seeding the cultivated cells to cell culture dishes after being passaged,
- in the meantime, amplifying the psPAX2 (lentiviral packaging plasmid), pMD2.G (lentiviral envelope plasmid) and pTGM2(V2) (expression plasmid) plasmids using *E.coli* bacteria,
- confirming the sequence of hTGM2 (V2) gene in pTGM2(V2),
- producing lentiviruses containing pTGM2 gene by delivering psPAX2, pMD2.G, pTGM2(V2) plasmids to HEK293T cells in triplets,
- when the mesenchymal stem cells (MSC) are at about 70% confluency, performing transduction process of the mesenchymal stem cells by adding lentiviruses onto the mesenchymal stem cells (MSC) in the culture dishes,
- incubating the mesenchymal stem cells (MSC) in the CO₂ incubator,
- at the end of the incubation, performing PBS washing and then seeding in a normal medium and proceeding with the usual cell culture,
- incubating the transduced cells in a medium containing Blasticidin antibiotic and eliminating the cells that has not taken up the transgene from the medium,
- proving that living cells have successfully acquired the gene,
- preparing poly(1,4-butylene succinate)/ poly-L-lactide (PLLA) (PBSu/PLLA) polymer tissue scaffolds by using salt leaching technique,
- seeding the cells onto the prepared PBSu/PLLA polymer tissue scaffolds,
- after waiting for adhesion of the cells to the tissue scaffolds, adding medium to the culture dishes,
- obtaining the nature identical cartilage tissue.

2. A production method for preparing nature identical cartilage tissue according to Claim 1, **characterized in that** the step of preparing PBSu/PLLA polymer tissue scaffolds by using salt leaching technique comprises the sub-steps of
- dissolving the poly(1,4-butylene succinate) (PBSu) and poly-L-lactide (PLLA) polymers in dichloromethane,
- mixing the prepared polymer solution with NaCl salts until it acquires a paste-like form and then pouring it into the glass petri dishes,
- one day later, taking the polymers out of the glass dishes and dialyzing them in distilled water,
- freezing and lyophilizing the polymers,
- cutting the polymers in the form of discs,
- sterilizing the cut discs in ethanol solution in the refrigerator,
- then washing with distilled water,
- pouring medium on the polymer in the dishes and incubating it in CO₂ incubator,
- then, upon discarding the medium, obtaining sterile tissue scaffold.

3. A production method for preparing nature identical cartilage tissue according to Claim 2, **characterized in that** the poly(1,4-butylene succinate) (PBSu) and poly-L-lactide (PLLA) polymers are weighed at a ratio of 1:1 and dissolved in dichloromethane to make 6% solutions.

4. A production method for preparing nature identical cartilage tissue according to Claim 2, **characterized in that** the prepared polymer solution is mixed with NaCl salts (300-500 microns in size) until it acquires a paste-like form and then poured into the 10 cm glass petri dishes coated with aluminum foil.

5. A production method for preparing nature identical cartilage tissue according to Claim 2, **characterized in that** the polymers are taken out of the glass dishes and dialyzed in distilled water for 2 days.

6. A production method for preparing nature identical cartilage tissue according to Claim 2, **characterized in that** the polymers are frozen and lyophilized at -80°C.

7. A production method for preparing nature identical cartilage tissue according to Claim 2, **characterized in that** the polymers are cut in the form of discs with a diameter of 7 mm.

8. A production method for preparing nature identical cartilage tissue according to Claim 2, **characterized in that** the cut discs are sterilized in 70% ethanol solution in the refrigerator for 2 hours.

9. A production method for preparing nature identical cartilage tissue according to Claim 2, **characterized in that** the sterilized discs are washed with distilled water at least three times.

10. A production method for preparing nature identical cartilage tissue according to Claim 2, **characterized in that** medium is poured on the polymer in the dishes and it is incubated in CO₂ incubator overnight.

11. **A** production method for preparing nature identical cartilage tissue according to Claim 1, **characterized in that** when the mesenchymal stem cells (MSC) are at about 70% confluency, adding the lentiviruses onto the mesenchymal stem cells (MSC) such that 2 ml of lentivirus is added per culture dish.

12. A production method for preparing nature identical cartilage tissue according to Claim 1, **characterized in that** the mesenchymal stem cells (MSC) are incubated in the CO₂ incubator for about 24 hours.

13. A production method for preparing nature identical cartilage tissue according to Claim 1, **characterized in that** the transduced cells are incubated in a medium containing Blasticidin antibiotic for 10 days.

## Patentansprüche

1. Ein Herstellungsverfahren zur Herstellung des naturidentischsten Knorpelgewebes zur Verwendung bei Knorpelgewebedefekten und Knorpelgeweberegeneration, **gekennzeichnet durch** die folgenden Schritte:
- Kultivierung der aus Rattenknochenmark gewonnenen mesenchymalen Stammzellen (MSC) in einer sterilen Umgebung in Zellkulturschalen,
- Aussäen der kultivierten Zellen in Zellkulturschalen nach deren Passage,
- zwischenzeitliche Amplifikation der Plasmide psPAX2 (lentiviralesVerpackungsplasmid), pMD2.G (lentiviralesHüllplasmid) und pTGM2(V2) (Expressionsplasmide) unter Verwendung von E.cali-Bakterien,
- Bestätigung der Sequenz vom hTGM2(V2)-Gen in pTGM2(V2),
- Herstellung von das pTGM2-Gen enthaltenden Lentiviren, indem die Plasmide psPAX2, pMD2.G, pTGM2(V2) in die HEK293T-Zellen in Triplets eingebracht werden,
- Durchführung des Transduktionsverfahrens der mesenchymalen Stammzellen durch Zugabe von Lentiviren zu den mesenchymalen Stammzellen (MSC) in den Kulturschalen, sobald die mesenchymalen Stammzellen (MSC) zu etwa 70% konfluent werden;
- Inkubation der mesenchymalen Stammzellen (MSC) im CO₂-Inkubator,
- am Ende der Inkubation, Waschen mit PBS und anschließendes Aussäen in ein normales Medium und Fortfahren mit der üblichen Zellkultur,
- Inkubation der transduzierten Zellen in einem Medium, das das Antibiotikum Blasticidin enthält, und Eliminierung derjenigen Zellen, die das Transgen nicht aus dem Medium aufgenommen haben,
- Nachweisen, dass die lebenden Zellen das Gen erfolgreich übernommen haben,
- Herstellung von Poly(1,4-Butylensuccinat)/Poly-L-Lactid (PLLA) (PBSu/PLLA) Polymer-Gewebegerüsten unter Verwendung der Salzauslaugungstechnik;
- Aussäen der Zellen auf die hergestellten PBSu/PLLA-Polymer-Gewebegerüste,
- Zugabe von Medium in die Kulturschalen, nach Abwarten der Adhäsion der Zellen an den Gewebegerüsten;
- Gewinnung des naturidentischen Knorpelgewebes.

2. Ein Herstellungsverfahren zur Herstellung von naturidentischem Knorpelgewebe nach Anspruch 1, **dadurch gekennzeichnet, dass** der Schritt der Herstellung von PBSu/PLLA-Polymer-Gewebegerüsten unter Verwendung der Salzauslaugungstechnik die folgenden Teilschritte umfasst
- Auflösen der Polymere Poly(1,4-butylensuccinat) (PBSu) und Poly-L-Lactid (PLLA) in Dichlormethan,
- Mischen der vorbereiteten Polymerlösung mit NaCl-Salzen, bis sie eine pastenartige Form annimmt, und anschließendes Ausgießen in die Glaspetrischalen,
- Entnommen der Polymere aus den Glasschalen und Dialysieren in destilliertem Wasser am darauffolgenden Tag,
- Einfrieren und Lyophilisieren der Polymere,
- Schneiden der Polymere in Form von Scheiben,
- Sterilisierung der geschnittenen Scheiben in Ethanollösung im Kühlschrank,
- AnschliessendesWaschen mit destilliertem Wasser,
- Ausgießen des Mediums auf das Polymer in den Schalen und Inkubieren im CO₂-Inkubator,
- AnschliessendeGewinnung vom sterilen Gewebegerüst nach Verwerfen des Mediums.

3. Ein Herstellungsverfahren zur Herstellung von naturidentischem Knorpelgewebe nach Anspruch 2, **dadurch gekennzeichnet, dass** die Polymere Poly(1,4-butylensuccinat) (PBSu) und Poly-L-Lactid (PLLA) im Verhältnis 1: 1 eingewogen und in Dichlormethan zu 6%igen Lösungen gelöst werden.

4. Ein Herstellungsverfahren zur Herstellung von naturidentischem Knorpelgewebe nach Anspruch 2, **dadurch gekennzeichnet, dass** die vorbereitete Polymerlösung mit NaCI-Salzen (300-500 Mikrometer Größe) gemischt wird, bis sie eine pastenartige Form annimmt, und dann in die mit Aluminiumfolie beschichteten 10-cm-Glaspetrischalen gegossen wird.

5. Ein Herstellungsverfahren zur Herstellung von naturidentischem Knorpelgewebe nach Anspruch 2, **dadurch gekennzeichnet, dass** die Polymere aus den Glasschalen entnommen und 2 Tage lang in destilliertem Wasser dialysiert werden.

6. Ein Herstellungsverfahren zur Herstellung von naturidentischem Knorpelgewebe nach Anspruch 2, **dadurch gekennzeichnet, dass** die Polymere bei -80°C eingefroren und lyophilisiert werden.

7. Ein Herstellungsverfahren zur Herstellung von naturidentischem Knorpelgewebe nach Anspruch 2, **dadurch gekennzeichnet, dass** die Polymere in Form von Scheiben mit einem Durchmesser von 7 mm geschnitten werden.

8. Ein Herstellungsverfahren zur Herstellung von naturidentischem Knorpelgewebe nach Anspruch 2, **dadurch gekennzeichnet, dass** die geschnittenen Scheiben in 70%iger Ethanollösung im Kühlschrank für 2 Stunden sterilisiert werden.

9. Ein Herstellungsverfahren zur Herstellung von naturidentischem Knorpelgewebe nach Anspruch 2, **dadurch gekennzeichnet, dass** die sterilisierten Scheiben mindestens dreimal mit destilliertem Wasser gewaschen werden.

10. Ein Herstellungsverfahren zur Herstellung von naturidentischem Knorpelgewebe nach Anspruch 2, **dadurch gekennzeichnet, dass** das Medium auf das Polymer in den Schalen gegossen und über Nacht im CO₂-Inkubator inkubiert wird.

11. Ein Herstellungsverfahren zur Herstellung von naturidentischem Knorpelgewebe nach Anspruch 1, **dadurch gekennzeichnet, dass** die Lentiviren derart auf die mesenchymalen Stammzellen (MSC) gegeben werden, dass 2 ml Lentivirus pro Kulturschale zugegeben werden, sobald die mesenchymalen Stammzellen (MSC) zu etwa 70 % konfluiert sind.

12. Ein Herstellungsverfahren zur Herstellung von naturidentischem Knorpelgewebe nach Anspruch 1, **dadurch gekennzeichnet, dass** die mesenchymalen Stammzellen (MSC) etwa für eine Dauer von 24 Stunden im CO₂-Inkubator inkubiert werden.

13. Ein Herstellungsverfahren zur Herstellung von naturidentischem Knorpelgewebe nach Anspruch 1, **dadurch gekennzeichnet, dass** die transduzierten Zellen füe eine Dauer von 10 Tagen in einem Medium mit dem Antibiotikum Blasticidin inkubiert werden.

## Revendications

1. Une méthode de production pour la préparation de tissus cartilagineux identiques à la nature de cartilage le plus identique possible à la nature, à utiliser dans les cas de défauts du tissu cartilagineux et de régénération du tissu cartilagineux, **caractérisée par** les étapes suivantes :
- cultivation des cellules souches mésenchymateuses (CSM) extraites de la moelle osseuse de rat dans un environnement stérile dans des boîtes de culture cellulaire,
- ensemencement des cellules cultivées dans des boîtes de culture cellulaire après leur passage,
- entre-temps, l'amplification du psPAX2 (plasmide d'emballage lentiviral), du pMD2.G (plasmide d'enveloppe lentiviral) et pTGM2(V2) (plasmide d'expression) en utilisant des bactéries E. coli,
- confirmation de la séquence du gène hTGM2 (V2) dans pTGM2(V2),
- production de lentivirus contenant le gène pTGM2 en délivrant les plasmides psPAX2, pMD2.G, pTGM2(V2) dans des cellules HEK293T en triplets,
- lorsque les cellules souches mésenchymateuses (CSM) ont atteint un taux de confluence d'environ 70 %, réalisation du processus de transduction des cellules souches mésenchymateuses en ajoutant des lentivirus sur les cellules souches mésenchymateuses (CSM) dans les boîtes de culture,
- l'incubation des cellules souches mésenchymateuses (CSM) dans l'incubateur à CO₂,
- à la fin de l'incubation, lavage au PBS et ensuite ensemencement dans un milieu normal et continuation de la culture cellulaire habituelle,
- incubation des cellules transduites dans un milieu contenant l'antibiotique Blasticidin et élimination du milieu des cellules qui n'ont pas absorbé le transgène,
- démontrant que les cellules vivantes ont réussi à acquérir le gène,
- la préparation d'échafaudages tissulaires en polymère poly(1,4-butylène succinate)/poly-L-lactide (PLLA) (PBSu/PLLA) en utilisant la technique de lixiviation par le sel,
- ensemencement des cellules sur les échafaudages préparés en polymère PBSu/PLLA,
- après avoir attendu l'adhésion des cellules aux échafaudages tissulaires, ajout de milieu dans les boîtes de culture,
- obtention du tissu cartilagineux le plus identique à la nature.

2. Une méthode de production pour préparer un tissu cartilagineux identique à la nature selon la revendication 1, **caractérisée par le fait que** l'étape de préparation des échafaudages de tissu polymère PBSu/PLLA en utilisant la technique de lixiviation par le sel comprend les sous-étapes suivantes :
- dissolution des polymères poly(1,4-butylène succinate) (PBSu) et poly-L-lactide (PLLA) dans du dichlorométhane,
- mélange de la solution de polymère préparée avec des sels de NaCl jusqu'à ce qu'elle prenne la forme d'une pâte, puis versement dans les boîtes de petri en verre,
- un jour plus tard, en retirant les polymères des boîtes en verre et en les dialysant dans de l'eau distillée,
- congélation et lyophilisation des polymères,
- découpe des polymères sous forme de disques,
- stérilisation des disques coupés dans une solution d'éthanol au réfrigérateur,
- puis lavage à l'eau distillée,
- versement de milieu sur le polymère dans les boîtes et incubation dans un incubateur à CO₂,
- puis, après élimination du milieu, obtention d'un échafaudage tissulaire stérile.

3. Une méthode de production pour préparer un tissu cartilagineux identique à la nature selon la revendication 2, **caractérisée par le fait que** les polymères poly(1,4-butylèn succinate (PBSu) et poly-L-lactide (PLLA) sont pesés dans un rapport de 1:1 et dissous dans du dichlorométhane pour obtenir des solutions à 6%.

4. Une méthode de production pour préparer un tissu cartilagineux identique à la nature selon la revendication 2, **caractérisée par le fait que** la solution de polymère préparée est mélangée avec des sels de NaCI (300-500 microns) jusqu'à ce qu'elle prenne la forme d'une pâte, puis versée dans des boîtes de petri en verre de 10 cm recouvertes d'une feuille d'aluminium.

5. Une méthode de production pour préparer un tissu cartilagineux identique à la nature selon la revendication 2, **caractérisée par le fait que** les polymères sont retirés des boîtes en verre et dialysés dans de l'eau distillée pendant 2 jours.

6. Une méthode de production pour préparer un tissu cartilagineux identique à la nature selon la revendication 2, **caractérisée par le fait que** les polymères sont congelés et lyophilisés à -80°C.

7. Une méthode de production pour la préparation de tissus cartilagineux identiques à la nature selon la revendication 2, **caractérisée par le fait que** les polymères sont coupés sous forme de disques d'un diamètre de 7 mm.

8. Une méthode de production de tissu cartilagineux identique à la nature selon la revendication 2, **caractérisée par le fait que** les disques coupés sont stérilisés dans une solution d'éthanol à 70 % dans le réfrigérateur pendant 2 heures.

9. Une méthode de production pour préparer un tissu cartilagineux identique à la nature selon la revendication 2, **caractérisée par le fait que** les disques stérilisés sont lavés avec de l'eau distillée au moins trois fois.

10. Une méthode de production pour préparer un tissu cartilagineux identique à la nature selon la revendication 2, **caractérisée par le fait que** le milieu est versé sur le polymère dans les boîtes et qu'il est incubé dans un incubateur à CO₂ pendant la nuit.

11. Une méthode de production pour préparer un tissu cartilagineux identique à la nature selon la revendication 1, **caractérisée par le fait que** lorsque les cellules souches mésenchymateuses (CSM) sont à environ 70 % de confluence, les lentivirus sont ajoutés aux cellules souches mésenchymateuses (CSM) de manière à ce que 2 ml de lentivirus soient ajoutés par boîte de culture.

12. Une méthode de production pour préparer un tissu cartilagineux identique à la nature selon la revendication 1, **caractérisée par le fait que** les cellules souches mésenchymateuses (CSM) sont incubées dans l'incubateur à CO₂ pendant environ 24 heures.

13. Une méthode de production pour préparer un tissu cartilagineux identique à la nature selon la revendication 1, **caractérisé par le fait que** les cellules transduites sont incubées dans un milieu contenant l'antibiotique Blasticidin pendant 10 jours.
